# EUROPÄISCHE PATENTSCHRIFT

(11) **EP 3 629 845 B1**
(45) Veröffentlichungstag und Bekanntmachung des Hinweises auf die Patenterteilung: **20.11.2024**
(21) Anmeldenummer: 18743672.0
(22) Anmeldetag: 26.05.2018
(51) Int. Cl.: A47C 31/12, A61B 5/00

(54) **MESSVORRICHTUNG**
MEASURING DEVICE
DISPOSITIF DE MESURE

(30) Priorität: 26.05.2017 DE 202017103200 U
(43) Veröffentlichungstag der Anmeldung: 08.04.2020
(73) Patentinhaber: Corvisu UG (Haftungsbeschränkt), 66346 Püttlingen (DE)
(72) Erfinder: RAUSCH, Daniel, 66346 Püttlingen (DE); PICHLMEIER, Florian, 81825 München (DE)
(74) Vertreter: Rausch, Daniel
(86) Internationale Anmeldenummer: PCT/DE2018/100514
(87) Internationale Veröffentlichungsnummer: WO 2018/215029

(56) Entgegenhaltungen:
- WO-A1-2009/038493
- FR-A1- 2 449 433
- GB-A- 2 262 810

## Beschreibung

Die Erfindung betrifft eine Messvorrichtung sowie ein Verfahren zur individuellen Anpassung einer Liegefläche.

Wie allgemein bekannt ist, leidet ein Großteil der Menschen insbesondere der Industrienationen aufgrund der geänderten Arbeitsbedingungen zumindest gelegentlich an Rückenschmerzen. Oftmals werden die Schmerzen dabei chronisch und schränken Lebensqualität und Leistungsfähigkeit immer weiter ein. Rückenschmerzen entstehen dabei auch durch schlechte Bettlagerung oder werden hierdurch zumindest verstärkt. Daher ist die Wahl des richtigen Schlafsystems von entscheidender Bedeutung bei der Vermeidung und Behandlung von Rückenschmerzen und fördert zudem einen erholsamen Schlaf.

Aus der DE 10 2011 119 039 B4 ist ein Verfahren zur Diagnostik eines Schlafsystems bekannt. Dabei wird mittels einer Kamera der Körper einer auf einem Schlafsystem ruhenden Person erfasst und mit einem weiteren Bild der stehenden Person überlagert. Mittels des so erstellten Überlagerungsbilds wird die Eignung des Schlafsystems für die betreffende Person beurteilt.

Aus FR 2 449 433 A1 ist eine Gliederkette zur Erfassung der Wirbelsäulenposition bekannt, bei welcher einzelne Kettenglieder jeweils über ein Kardangelenk verbunden sind.

Aufgabe der Erfindung ist es, eine Messvorrichtung zu schaffen, welche eine besonders einfache und präzise Bestimmung der Positionierung der menschlichen Wirbelsäule ermöglicht. Zudem soll ein Verfahren geschaffen werden, welches eine individuelle Anpassung einer Liegefläche an eine Person ermöglicht.

Diese Aufgabe wird durch eine Messvorrichtung mit den Merkmalen des Anspruchs 1 sowie ein Verfahren mit den Merkmalen des Anspruchs 9 gelöst. Vorteilhafte Ausgestaltungen der Erfindung sind in den Unteransprüchen angegeben.

Die erfindungsgemäße Messvorrichtung zeichnet sich dadurch aus, dass sie eine Gliederkette mit mehreren gelenkig verbundenen Kettengliedern umfasst, wobei jedes Kettenglied einen Kopplungsabschnitt zur Verbindung mit einem benachbarten Kettenglied aufweist und jeder Kopplungsabschnitt jeweils mehrere Gelenke mit Winkel- und/oder Längensensoren zur Bestimmung der relativen gegenseitigen Position benachbarter Kettenglieder aufweist. Hierdurch wird eine besonders einfache Messvorrichtung geschaffen, welche eine sehr präzise Bestimmung der Positionierung der menschlichen Wirbelsäule ermöglicht.

Erfindungsgemäß umfasst die Messvorrichtung einen Schwerkraftsensor zur Bestimmung der räumlichen Orientierung. Durch den Schwerkraftsensor kann die räumliche Orientierung desjenigen Kettenglieds, an dem der Schwerkraftsensor montiert ist, ermittelt werden. Über die bereits beschriebenen Drehwinkel- und/oder Längensensoren kann hiervon ausgehend die Lage jedes einzelnen Kettenglieds und damit die Lage der gesamten Messvorrichtung im Raum bestimmt werden. Dies ermöglicht neben der Bestimmung der Positionierung der Wirbelsäule im Raum auch die Orientierung im Raum und damit die Einstellung einer bevorzugt horizontalen Wirbelsäulenorientierung, was für das Wohlbefinden einer auf einer Liegefläche liegenden Testperson ebenso wichtig ist.

Zur Erfassung der relativen Winkellage zwischen benachbarten Kettengliedern kann vorgesehen sein, dass jeder Kopplungsabschnitt jeweils drei Drehgelenke mit zugeordneten Winkelsensoren aufweist. Dabei können beispielsweise zwei Drehgelenke jeweils als Kardangelenk zusammengefasst sein. Mittels dieser drei Drehgelenke an jedem Kopplungsabschnitt kann die Gliederkette die Positionierung der Wirbelsäule, also die Flexion/Retroflexion, die Lateralflexion und die Torsion, besonders präzise nachbilden, so dass über die zugeordneten Winkelsensoren die Positionierung exakt bestimmt werden kann.

In einer vorteilhaften Ausführungsform weist jeder Kopplungsabschnitt zusätzlich ein Schubgelenk mit einem Längensensor auf. Das Schubgelenk ist dabei derart orientiert, dass es zumindest eine Bewegungskomponente in Längsrichtung der Gliederkette umfasst, also entlang der miteinander verbundenen Kettenglieder. Hierdurch kann die Gliederkette in Längsrichtung gestreckt oder gestaucht werden, was eine besonders gute Anpassung an die zu vermessende Wirbelsäule in den verschiedenen Positionierungen ermöglicht. Durch die Erfassung der variablen Abstände zwischen den Kettengliedern mittels der Längensensoren wird zudem eine besonders präzise Berechnung der tatsächlichen Positionierung der Wirbelsäule ermöglicht.

Eine besonders gute und einfache Anpassung der Messvorrichtung an die Wirbelsäule kann dabei dadurch erreicht werden, dass ein Drehgelenk jedes Kopplungsabschnitts mittels eines Federelements vorgespannt ist. Bei diesen Drehgelenken handelt es sich bevorzugt um die Drehgelenke zur Erfassung der Flexion/Retroflexion der Wirbelsäule. So können beispielsweise die jeweiligen Drehgelenke mittels einer Drehfeder gegen einen Anschlag, welcher eine Drehwinkelbegrenzung bildet, beaufschlagt sein. Zudem können die jeweiligen Drehgelenke alle in die gleiche Richtung vorgespannt werden, wodurch sich ein U-förmiger Verlauf der Gliederkette ergibt, oder abschnittsweise in entgegengesetzter Richtung vorgespannt sein, wodurch beispielsweise ein S-förmiger Verlauf der Gliederkette erreicht wird. Hierdurch kann erreicht werden, dass die Gliederkette nur an wenigen Stellen beispielsweise mittels Spannbändern oder einem textilen Grundkörper gegen die Wirbelsäule der Testperson beaufschlagt werden muss und die übrigen Elemente sich gegenseitig gegen die Wirbelsäule andrücken. Bei einem eingestellten U-förmigen Verlauf kann die Messvorrichtung beispielsweise derart zur Wirbelsäule positioniert werden, dass die Enden der Gliederkette von der Wirbelsäule abstehen. Werden nun die Enden der Gliederkette gegen die Wirbelsäule beaufschlagt, werden ohne Weiteres auch die dazwischen liegenden Kettenglieder gegen die Wirbelsäule beaufschlagt, wodurch jedes Kettenglied dauerhaft gegenüber der Wirbelsäule positioniert bleibt. Ebenso könnte eine U-förmig verlaufende Gliederkette auch mit den Enden zur Wirbelsäule positioniert und beispielsweise mittels eines einzigen Spannbands in der Mitte der Gliederkette oder einem textilen Grundkörper hiermit über die gesamte Länge gegen die Wirbelsäule beaufschlagt werden. Durch die Anpassung und Orientierung der Federkraft der Federelemente kann dabei besonders einfach die Form der Messvorrichtung und die Haltekraft gegenüber der Wirbelsäule eingestellt werden. Insbesondere können die Federvorspannungen der Federelemente der einzelnen Koppelungsabschnitte unterschiedlich eingestellt sein, um eine bestimmte Haltekraftverteilung einzustellen.

Um eine möglichst einfache Handhabung des Messvorrichtung zu gewährleisten, kann ein Datenübertragungsmodul, insbesondere ein Funk-Übertragungsmodul, zur Übertragung der Daten der Sensoren von der Gliederkette beispielsweise an eine Auswerte- und Anzeigeeinheit vorgesehen sein. Durch die Datenübertragung per Funk kann die Messvorrichtung frei bewegt werden, was insbesondere bei einer liegenden Testperson vorteilhaft ist. Zudem kann über die Funkübertragung eine universelle Verbindung zu einer sehr große Auswahl an Auswerte- und Anzeigeeinheiten hergestellt werden. Beispielsweise können hierbei über WLAN oder Bluetooth ^{®} auch Mobiltelefone oder Tablets als Auswerte- und Anzeigeeinheit verwendet werden.

Zudem kann die Handhabung der Messvorrichtung dadurch erleichtert werden, dass diese einen Energiespeicher zur autonomen Energieversorgung der Gliederkette umfasst. Hierbei kann es sich beispielsweise um eine Batterie oder einen Akkumulator handeln, welche über einen Schalter zum Aktivieren und Deaktivieren mit einer Erfassungseinheit der Sensoren verbindbar sind.

Zudem wird auch ein Bekleidungsstück zur Messung der Positionierung des menschlichen Oberkörpers beansprucht, welches dadurch gekennzeichnet ist, dass es die zuvor beschriebene Messvorrichtung umfasst. Durch die Integration der Messvorrichtung in ein Bekleidungsstück wird ein besonders einfaches Anlegen der Messvorrichtung an die Testperson ermöglicht. Zudem kann über entsprechende Justiervorrichtungen eine lagegenaue Positionierung der Messvorrichtung entlang der Wirbelsäule der Testperson erfolgen. Beispielsweise kann über optisch abgesetzte Streifen in dem Bekleidungsstück der Verlauf der Messvorrichtung in dem Kleidungsstück kenntlich gemacht sein, so dass eine einfache Verschiebung des Kleidungsstücks und damit Anordnung der Messvorrichtung direkt über der Wirbelsäule erleichtert wird.

Um ein einfaches Austauschen der Messvorrichtung beispielsweise zur Reinigung des Bekleidungsstücks zu ermöglichen kann die Messvorrichtung mittels einer lösbaren Befestigungsvorrichtung an dem Grundkörper befestigt sein. Es ist jedoch auch möglich, die Messvorrichtung wasserdicht auszubilden, so dass diese zusammen mit dem Bekleidungsstück gereinigt werden kann.

Um eine eigenständige Anpassung der Messvorrichtung in dem Bekleidungsstück an die Wirbelsäule durch die Testperson zu ermöglichen, kann die Justiervorrichtung auch mindestens ein Paar von Bändern aufweisen, welche sich auf gleicher Höhe, also von einem bestimmten Kettenglied der Gliederkette zu beiden Seiten um den Torso der Testperson erstrecken. Bevorzugt sind jedoch eine Mehrzahl derartiger Paare von Bändern über die Längserstreckung der Messvorrichtung verteilt an dem Bekleidungsstück angeordnet, um die Messvorrichtung an mehreren Stellen gegenüber der Wirbelsäule justieren zu können. Die Länge der Bänder ist dabei so gewählt, dass diese jeweils von der Messvorrichtung, also einem bestimmten Kettenglied, am Rücken der Testperson zu beiden Seiten um den Torso seitlich herumreichen und sich bevorzugt im Brust-Bauchbereich überlappen. Das Paar von Bändern weist eine bestimmte Länge auf, ist also zumindest in Längsrichtung nicht elastisch, so dass die Testperson über ein Ziehen an jeweils einem der Bänder des Paars ein seitliches Verschieben der Messvorrichtung erreichen kann. Um eine mittige Zentrierung der Messvorrichtung über der Wirbelsäule zu erreichen, können an jedem Band (Strich-)Markierungen mit beispielsweise verschiedenen Farben ausgebildet sein, welche jeweils bestimmte Abstände von der Messvorrichtung kennzeichnen. Je nach Umfang des Torsos kann daher die Testperson das Paar an Bändern im Brust-Bauchbereich zusammenführen und die Bänder anschließend derart seitlich verschieben, dass der Überlappungsbereich einer identischen Markierung, welche einen gleichen Abstand zu beiden Richtungen zur Messvorrichtung am Rücken kennzeichnet, an der Vorderseite mittig ausgerichtet ist. Hierdurch kann die Testperson ohne die Zuhilfenahme einer Hilfsperson oder eines Spiegels sehr einfach das Bekleidungsstück mit der Messvorrichtung gegenüber der Wirbelsäule justieren.

Als Bekleidungsstück sind die üblichen Oberbekleidungen zu verstehen, also ein Kleidungsstück mit Hals- und Armöffnungen, welches bevorzugt eine hohe Elastizität aufweist.

Zudem werden eine Vorrichtung und ein Verfahren zur individuellen Anpassung einer Liegefläche beansprucht. Die Vorrichtung umfasst dabei ein anpassbares Schlafsystem, eine Auswerteeinheit und die oben beschriebene Messvorrichtung bzw. das oben beschriebene Bekleidungsstück mit der Messvorrichtung. Das Schlafsystem weist eine Liegefläche auf, auf welche die Testperson sich hinlegen kann. In der einfachsten Version umfasst das Schlafsystem eine Schlafmatratze, welche an Ihrer Oberseite die Liegefläche aufweist. Zudem kann das Schlafsystem aber auch einen Unterbau, insbesondere einen Lattenrost, und/oder ein Kopfkissen umfassen.

Das Schlafsystem kann derart anpassbar ausgebildet sein, dass beispielsweise lediglich das Kopfkissen gegenüber der Matratze verschiebbar ist. Alternativ oder ergänzend kann auch die Matratze als Ganzes austauschbar sein oder in Ihrer Härte gesamt oder abschnittsweise verstellbar sein, was beispielsweise bei Luft- oder Wassermatratzen über den Druck regelbar ist. Alternativ oder ergänzend kann zudem ein verstellbarer Lattenrost vorgesehen sein, mit Hilfe dessen die Einsinktiefe der Matratze in bekannter Art durch Veränderung der Lattenhärte abschnittsweise eingestellt werden kann.

Gemäß dem Verfahren wird dabei zur Anpassung der Liegefläche zuerst die Messvorrichtung bzw. das Bekleidungsstück mit der Messvorrichtung an einer Testperson angebracht, beispielsweise indem die Testperson das Bekleidungsstück anzieht und gegebenenfalls auch wie oben beschrieben justiert, um die Messvorrichtung exakt zur Wirbelsäule zu positionieren. Anschließend wird die Ist-Positionierung der Wirbelsäule der auf der Liegefläche ruhenden Testperson ermittelt. Diese kann beispielsweise anschließend auch mit einer Soll-Positionierung aus hinterlegten Daten verglichen werden. Je nach dem Ergebnis der Ist-Positionierung der Wirbelsäule kann anschließend die Auswahl und Anpassung der Liegefläche in Abhängigkeit der Soll-Positionierung der Wirbelsäule erfolgen. Dabei kann das anpassbare Schlafsystem wie oben beschrieben verändert werden, um bei der Wirbelsäule der Testperson die Soll-Positionierung zu erreichen.

Besonders vorteilhaft kann das anpassbare Schlafsystem eine automatische Einstellvorrichtung aufweisen, welche in Abhängigkeit der gemessenen Ist-Positionierung der Wirbelsäule der Testperson eine automatische Anpassung des Schlafsystems ermöglicht. Die Einstellvorrichtung kann dabei beispielsweise einen automatischen Lattenrost aufweisen, in welchem einzelne Latten oder Bereiche von Latten in ihrer Härte automatisch verstellbar sind, beispielsweise mittels Stellmotoren. Alternativ oder ergänzend kann auch die Härte der Matratze entsprechend automatisch einstellbar sein. Hierdurch kann besonders einfach eine individuelle Anpassung des Schlafsystems und damit der Liegefläche erfolgen. Zusätzlich kann auch vorgesehen sein, dass die Messvorrichtung über einen Großteil der Schlafdauer oder über die gesamte Schlafdauer die Ist-Positionierung der Wirbelsäule erfasst und entsprechend eine Anpassung des Schlafsystems durch die Einstellvorrichtung vorgenommen wird.

Zur Visualisierung der Ist-Positionierung der Wirbelsäule kann auch eine Anzeigeeinheit vorgesehen sein, welche die von der Auswerteeinheit ermittelten Daten anzeigt, beispielsweise indem ein 3D-Modell einer Wirbelsäule positionsgetreu dargestellt wird. In einer besonders bevorzugten Ausführungsform wird das 3D-Modell der Wirbelsäule dabei in einer dynamischen Seitenansicht gezeigt, welche dadurch gekennzeichnet ist, dass der durch den Schwerkraftsensor ermittelte Schwerkraftvektor parallel zur Bildebene angeordnet ist. Hierdurch wird erreicht, dass die optische Darstellung des 3D-Modells immer von der Seite zu erkennen ist, so dass der Einfluss der Matratze, welche im Wesentlichen vertikal in Richtung des Schwerkraftvektors abstützt, direkt ersichtlich ist. Die zweite Richtungskomponente der Bildebene der Seitenansicht kann dabei durch die Verbindungslinie zweier Kettenglieder der Gliederkette konstruiert werden, bevorzugt einem ersten Kettenglied und einem letzten Kettenglied. Die Blickrichtung der Seitenansicht ergibt sich damit aus dem Kreuzprodukt des Schwerkraftvektors und der beschriebenen Verbindungslinie. Eine derartige dynamische Seitenansicht wird automatisch und in Echtzeit angepasst und ermöglicht so die direkte Überprüfung der vorgenommenen Änderungen an dem anpassbaren Schlafsystem.

Weitere Besonderheiten und Vorzüge der Erfindung ergeben sich aus der folgenden Beschreibung bevorzugter Ausführungsbeispiele anhand der Zeichnungen. Es zeigen:
- **Fig. 1**: eine erste schematische Seitenansicht auf eine liegende Testperson, wie dies in der Druckschrift DE 10 2011 119 039 B4 gezeigt ist;
- **Fig. 2**: eine zweite schematische Seitenansicht auf eine liegende Testperson, wie dies in der Druckschrift DE 10 2011 119 039 B4 gezeigt ist;
- **Fig. 3**: eine erste perspektivische Ansicht auf eine erfindungsgemäße Messvorrichtung mit einer Gliederkette, welche entlang einer freigestellten Wirbelsäule einer Testperson angeordnet ist;
- **Fig. 4**: eine Draufsicht auf die Messvorrichtung von Figur 3 mit einer geradlinig angeordneten Gliederkette;
- **Fig.5**: eine Rückansicht auf ein Bekleidungsstück mit der Messvorrichtung von Figur 4 und
- **Fig. 6**: eine perspektivische Ansicht auf das Bekleidungsstück von Figur 5.

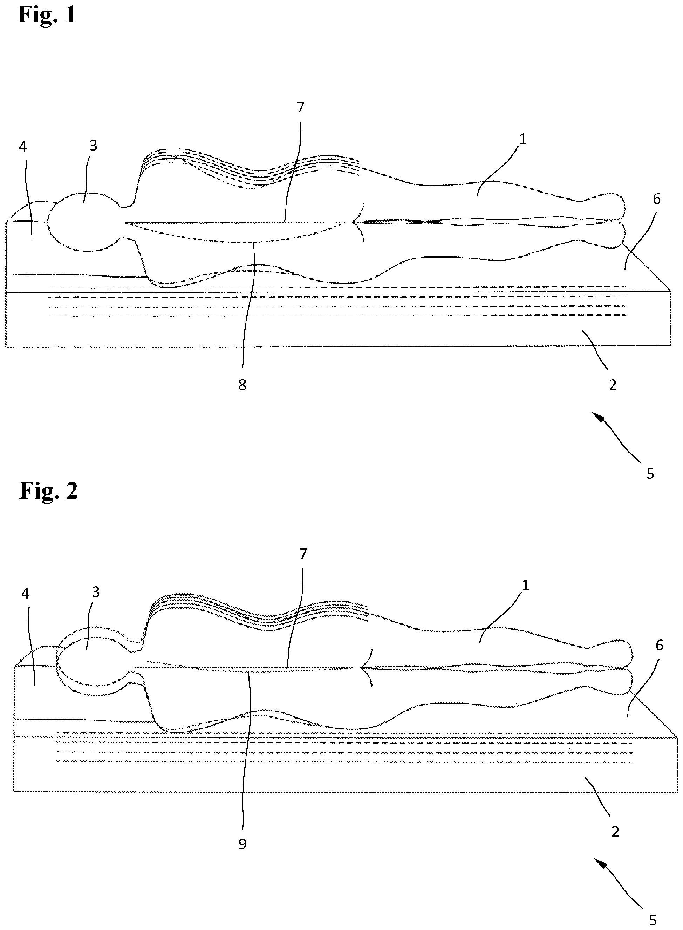

In Figur 1 ist eine erste schematische Seitenansicht auf eine Testperson 1 gezeigt, welche in der Seitenlage auf einer Matratze 2 liegt. Zusätzlich wird der Kopf 3 der Testperson 1 über ein Kopfkissen 4 abgestützt. Bei dieser Ausgestaltung bilden das Kopfkissen 4 und die Matratze 2 ein Schlafsystem 5 mit einer nach oben gewandten Liegefläche 6. In einer nicht dargestellten Ausführungsform kann unterhalb der Matratze 2 beispielsweise auch ein Lattenrost oder ein Bettgestell ausgebildet und von dem Schlafsystem umfasst sein.

Wie der Darstellung von Figur 1 zu entnehmen ist, ist die Testperson 1 dort mit zwei unterschiedlichen Körperhaltungen dargestellt. In einer ersten Körperhaltung, welche mit durchgezogenen Linien im Bereich des Oberkörpers gezeigt ist, ist die Wirbelsäule 7 der Testperson 1 in der Seitenansicht horizontal angeordnet, also besonders rückenschonend gelagert. Im Gegensatz hierzu zeigt die zweite Köperhaltung, welche mit gestrichelten Linien im Bereich des Oberkörpers dargestellt ist, die Auswirkung einer zu weichen Lagerung durch die Matratze 2. Hierbei wird die Wirbelsäule 8 zwischen den Schultern und dem Becken nicht ausreichend gestützt und hängt durch, was bei längerer derartiger Lagerung zu Schmerzen in der gekrümmten Wirbelsäule 8 führen kann.

In Figur 2 ist eine zweite schematische Seitenansicht auf die Testperson 1 von Figur 1 gezeigt. Dieselben Elemente sind mit den gleichen Bezugszeichen versehen. Im Unterschied zur Darstellung von Figur 1 ist hier der Vergleich zwischen der gewünschten horizontalen Lagerung der Wirbelsäule 7 zu einer gestrichelt dargestellten gekrümmten Lagerung der Wirbelsäule 9 gezeigt, welche entsteht, wenn die Lagerung durch die Matratze 2 im Bereich der Schultern zu hart ist, also nicht ausreichend einfedert. Auch hierdurch entstehen Spannungen in der gekrümmten Wirbelsäule 9, was bei längerer derartiger Lagerung ebenfalls zu Schmerzen führt.

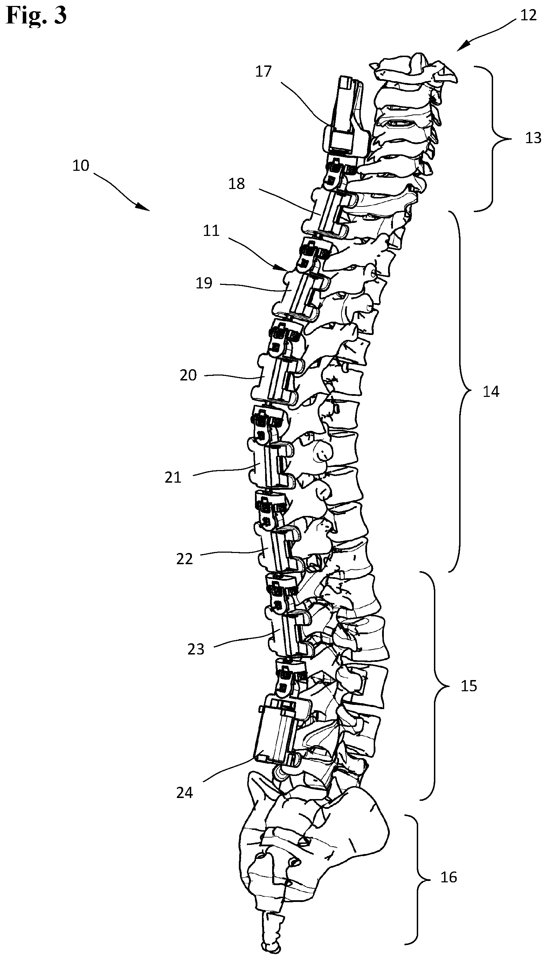

In Figur 3 ist eine erste perspektivische Ansicht auf eine erfindungsgemäße Messvorrichtung 10 mit einer Gliederkette 11 gezeigt, welche entlang einer freigestellten Wirbelsäule 12 einer Testperson angeordnet ist. Die Wirbelsäule 12 wird medizinisch in die Abschnitte Halswirbel 13, Brustwirbel 14, Lendenwirbel 15 und Kreuzbein/Steißbein 16 unterteilt.

Die Gliederkette 11 umfasst dabei mehre gelenkig verbundene Kettenglieder 17, 18, 19, 20, 21, 22, 23, 24, welche dem Verlauf der Wirbelsäule 12 mit geringem Abstand folgen und damit der Verlauf der Wirbelsäule 12 durch die Gliederkette 11 abgebildet wird. Die Gliederkette 11 erstreckt sich dabei über die Länge der Wirbelsäule 12 zumindest von den Halswirbeln 13 bis zu den Lendenwirbeln 15. Es kann auch vorgesehen sein, dass erste Ketteglied 17 am Kopf der Testperson und/oder das letzte Kettenglied 24 am Kreuzbein zu befestigen. Über die von der Rückseite der Wirbelsäule 12 abstehenden Dornfortsätze können die Kettenglieder 17, 18, 19, 20, 21, 22, 23, 24 der Gliederkette 11 beispielsweise mittels korrespondierender Aussparungen und/oder Füße exakt zur Wirbelsäule 12 gehalten und zentriert werden.

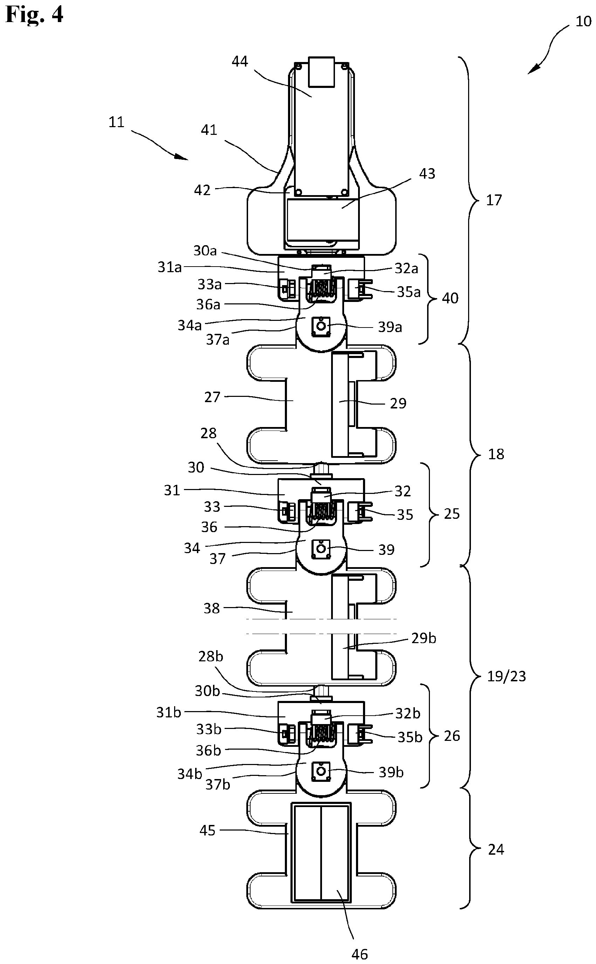

In Figur 4 ist eine Draufsicht auf die Messvorrichtung 10 von Figur 3 mit einer geradlinig angeordneten Gliederkette 11 gezeigt. Zur einfacheren Darstellung ist die Gliederkette 11 nicht in der gesamten Länge mit allen Kettengliedern 17, 18, 19, 20, 21, 22, 23, 24 gezeigt, sondern im Bereich des dritten Kettenglieds 19 zeichnerisch unterbrochen. Das vierte Kettenglied 20, fünfte Kettenglied 21, sechste Kettenglied 22 und siebte Kettenglied 23 sind nicht weiter dargestellt, jedoch identisch zu dem zweiten Kettenglied 18 und dem dritten Kettenglied 19 ausgebildet, so dass die Beschreibung zu den Kettengliedern 18 und 19 auch auf die nicht dargestellten Kettenglieder 20, 21, 22 und 23 zutrifft.

Wie der Figur 4 zu entnehmen ist, weist das zweite Kettenglied 18 einen Kopplungsabschnitt 25 zur gelenkigen Verbindung mit dem benachbarten Kettenglied 19 auf. Zudem weist auch das dritte Kettenglied 19 einen entsprechenden Kopplungsabschnitt auf, welcher aufgrund der unterbrochenen Darstellung mit den Bruchkanten im Bereich des dritten Kettenglieds 19 nicht dargestellt ist, jedoch anhand des dargestellten Kopplungsabschnitts 26 des siebten Kettenglieds 23 exemplarisch beschrieben wird.

Nachfolgend wird die gelenkige Verbindung zwischen den Kettengliedern 18-23 am Beispiel des Kopplungsabschnitts 25 zwischen dem zweiten Kettenglied 18 und dem dritten Kettenglied 19 beschrieben. Der Kopplungsabschnitt 25 weist am Übergang zu einem Grundkörper 27 des zweiten Kettenglieds 18 ein Schubgelenk 28 auf, welches eine Verschiebung des Kopplungsabschnitts 25 relativ zum Grundköper 27 in Längsrichtung der Gliederkette 11, also in der Darstellung von Figur 4 in vertikaler Richtung, ermöglicht. Zur Erfassung der Längsverschiebung ist das Schubgelenk 28 in dem Grundkörper 27 gelagert und mit einem Längensensor 29 in Form eines Schiebepotentiometers ausgebildet.

Die Schubstange des Schubgelenks 28 ist wiederum mittels eines ersten Drehgelenks 30, dessen Rotationsachse in Längsrichtung der Gliederkette 11 orientiert ist, mit einem ersten Zwischenstück 31 des Kopplungsabschnitts 25 verbunden. In Längsrichtung des ersten Drehgelenks 30 ist in dem ersten Zwischenstück 31 ein erster Winkelsensor 32 zur Erfassung des relativen Drehwinkels zwischen der Schubstange des Schubgelenks 28 und dem ersten Zwischenstück 31 angeordnet und mit der Schubstange des Schubgelenks 28 verbunden. Der Winkelsensor 32 ist als Drehpotentiometer ausgebildet.

In dem ersten Zwischenstück 31 ist ein zweites Drehgelenk 33 angeordnet, welches das erste Zwischenstück 31 mit einem zweiten Zwischenstück 34 des Kopplungsabschnitts 25 drehbar verbindet. Die Rotationsachse des zweiten Drehgelenks 33 ist rechtwinklig zur Rotationsachse des ersten Drehgelenks 30 und zudem parallel zu einer gedachten Anlagefläche am Rücken der Testperson orientiert, so dass durch ein am zweiten Drehgelenk 33 angeordneten zweiten Winkelsensor 35 die Flexion/Retroflexion einer Wirbelsäule bestimmbar ist. Der Winkelsensor 35 ist ebenfalls als Drehpotentiometer ausgebildet.

Um das Drehgelenk 33 zwischen dem ersten Zwischenstück 31 und dem zweiten Zwischenstück 34 um die Rotationsachse betreffend die Flexion/Retroflexion einer Wirbelsäule in eine bestimmte Winkellage zu beaufschlagen, ist ein Federelement 36 vorgesehen, welches das zweite Drehgelenk 33 gegen einen definierten Winkelanschlag vorspannt. Hierdurch kann die bereits beschriebene Verformung der Gliederkette 11 beispielsweise in U-Form oder S-Form erreicht werden, welche einen besonders guten Andruck der Gliederkette 11 an die Wirbelsäule bzw. die Dornfortsätze der Wirbelsäule ermöglicht.

In dem zweiten Zwischenstück 34 ist ein drittes Drehgelenk 37 gelagert, welches das zweite Zwischenstück 34 drehbar mit einem Grundkörper 38 des dritten Kettenglieds 19 verbindet. Die Rotationsachse des dritten Drehgelenks 37 ist rechtwinklig zur Rotationsachse des ersten Drehgelenks 30 und zudem rechtwinklig zur Rotationsachse des zweiten Drehgelenks 33 ausgebildet. Zur Bestimmung des Drehwinkels des dritten Drehgelenks 37 ist ein ebenfalls als Drehpotentiometer ausgebildeter dritter Winkelsensor 39 vorgesehen. Über die richtwinklig zueinander angeordneten Rotationsachsen der Drehgelenke 30, 33, 37 wird damit ein Freiheitsgrad zwischen den Kettengliedern ähnlich einer Kardanwelle erreicht, welche auch in einer alternativen Ausführungsform verwendet werden kann.

Die gelenkige mechanische Verbindung zwischen den Kettengliedern 18-23 erfolgt damit in den jeweiligen Kopplungsabschnitten identisch zum Kopplungsabschnitt 25 mit den Drehgelenken 30, 33, 37 und dem Schubgelenk 28 über drei Drehgelenke und ein Schubgelenk. Durch die Ausbildung der Winkelsensoren und des Längensensors als Potentiometer kann besonders einfach eine digital auswertbare Spannungsteilerschaltung realisiert werden zur Erfassung und Auswertung der Winkel- und Längenwerte umgesetzt werden. Hieraus lässt sich schließlich der Verlauf der Gliederkette 11 berechnen und der Verlauf der Wirbelsäule ableiten. Vorteilhaft kann der Verlauf der Wirbelsäule durch einen Interpolation, insbesondere eine kubische Spline-Interpolation des Verlaufs der Gliederkette 11 bestimmt werden, was einen sehr genauen Rückschluss auf die Positionierung eines jeden Wirbels der Wirbelsäule und damit mögliche Fehlstellungen erlaubt.

Wie der Figur 4 weiter zu entnehmen ist, weisen der Grundkörper 27 des zweiten Kettenglieds 18 und der Grundkörper 38 des dritten Kettenglieds 19 wie auch die übrigen Kettenglieder 18-23 seitlich abstehende vier Füße auf, welche sich parallel zu einer Anlagefläche am Rücken der Testperson, welche parallel zur Bildebene von Figur 4 orientiert ist, erstrecken. Hierdurch wird eine besonders gute Anlage der Grundkörper der Kettenglieder 18-23 an den Rücken und die Wirbelsäule der Testperson und damit eine genaue Messung ermöglicht

Im Gegensatz zu den Kettengliedern 18-23 sind das erste Kettenglied 17 und das letzte, achte Kettenglied 24 geringfügig unterschiedlich ausgebildet. Das erste Kettenglied 17 weist einen Kopplungsabschnitt 40 auf, welcher im Wesentlichen identisch zu dem Kopplungsabschnitt 25 ausgebildet ist, wobei hier aus Platzgründen kein Schubgelenk mit einem Längensensor vorgesehen ist. Die im Übrigen identisch ausgebildeten weiteren Elemente sind mit den entsprechenden Bezugszeichen mit dem Zusatz a gekennzeichnet.

In einem Grundkörper 41 des Kettenglieds 17 sind ein Schwerkraftsensor 42, ein Funk-Datenübertragungsmodul 43 und eine Recheneinheit 44 angeordnet. Mittels der Recheneinheit 44 werden die Daten der Winkelsensoren und Längensensoren sowie des Schwerkraftsensors 42 erfasst und über das Funk-Datenübertragungsmodul 43 an eine Auswerte- und/oder Anzeigeeinheit weitergeleitet.

Zur Energieversorgung der Messvorrichtung 10 weist das achte Kettenglied 24 in einem Grundkörper 45 einen elektrochemischen Energiespeicher 46 auf. Da es sich bei dem achten Kettenglied 24 um das letzte Element der gezeigten Gliederkette 11 handelt, ist hier kein weiterer Kopplungsabschnitt vorgesehen. Mit dem siebten Kettenglied 23 ist das achte Kettenglied 24 über die gezeigte Kopplungsvorrichtung 26 gelenkig mechanisch verbunden, welche die gleichen Elemente wie die Kopplungsvorrichtung 25 des zweiten Kettenglieds 18 aufweist und deshalb mit den entsprechenden Bezugszeichen mit dem Zusatz b gekennzeichnet ist.

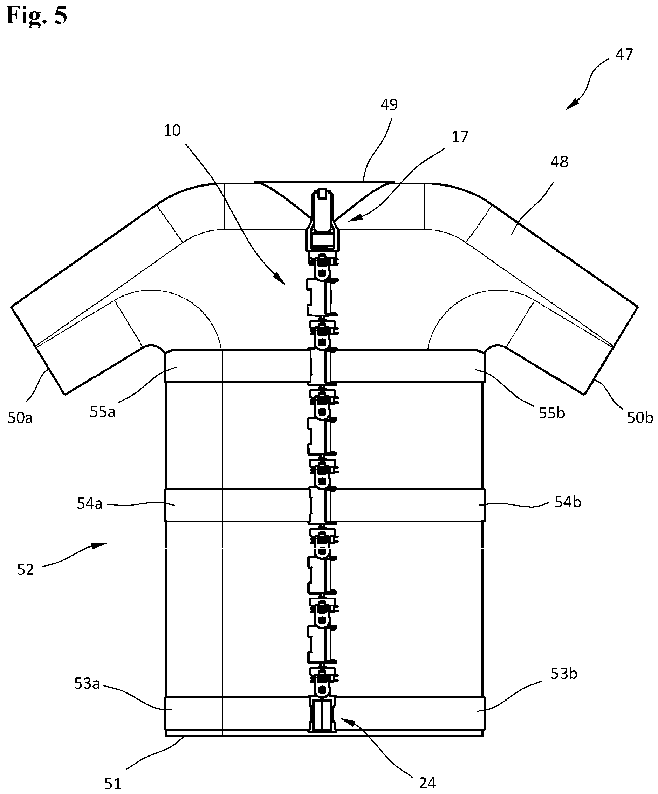

In Figur 5 ist eine Rückansicht auf ein Bekleidungsstück 47 mit der Messvorrichtung 11 von Figur 3 gezeigt. Das Bekleidungsstück 47 umfasst einen textilen Grundkörper in Form eines Hemds 48 mit einer Halsöffnung 49, zwei Armöffnungen 50a, 50b und einer Torsoöffnung 51. Bei dem Hemd 48 kann es sich beispielsweise um ein (Sport-)Unterhemd mit zumindest abschnittsweise geschlossener oder durchgehend offener, jedoch verschließbarer Vorderseite handeln. Bevorzugt weist das Hemd einen hohen Anteil elastischer Fasern auf, um eine besonders gute Anpassung an den Oberkörper der Testperson zu erreichen.

An der Rückseite des Hemds 48 ist die bereits beschriebene Messvorrichtung 10 lösbar befestigt, beispielsweise über nicht dargestellte lösbare Halteelemente. Vorteilhaft ist die Messvorrichtung 10 dabei derart in das Hemd 48 integriert, dass das Hemd 48 die Messvorrichtung 10 gegen den Körper und damit die Wirbelsäule der Testperson drückt. Dies kann dadurch erreicht werden, dass die Messvorrichtung zumindest mit den bereits beschriebenen Füßen zur Anlage an einem Rücken der Testperson innerhalb des Hemds 48, also an einer Innenseite des Hemds 48 angeordnet ist.

Wie der Figur 5 weiter zu entnehmen ist, erstreckt sich die Gliederkette 11 der Messvorrichtung 10 im Wesentlichen über die gesamte Höhe des Hemds 48, also von der Halsöffnung 49, an welche sich das erste Kettenglied 17 anschließt, bis zur Torsoöffnung 51, an welcher das letzte, achte Kettenglied 24 angeordnet ist. Hierdurch wird gewährleistet, dass die Messkette zuverlässig entlang der Wirbelsäule von den Halswirbeln bis zu den Lendenwirbeln bzw. dem Kreuzbein positioniert ist.

Zur Zentrierung der Gliederkette 11 über der Wirbelsäule ist eine Justiervorrichtung 52 vorgesehen, welche mehrere Paare von Bändern 53a, 53b, 54a, 54b, 55a, 55b umfasst. Jedes Paar von Bändern 53a, 53b, 54a, 54b, 55a, 55b erstreckt sich dabei ausgehend von einem Kettenglied zu beiden Seiten um den Brustbereich herum zu einer Vorderseite des Hemds 48. In dem gezeigten Ausführungsbeispiel ist an dem dritten Kettenglied 19 das Paar von Bändern 55a, 55b, an dem fünften Kettenglied 21 das Paar von Bändern 54a, 54b und an dem achten Kettenglied 24 das Paar von Bändern 53a, 53b angeordnet.

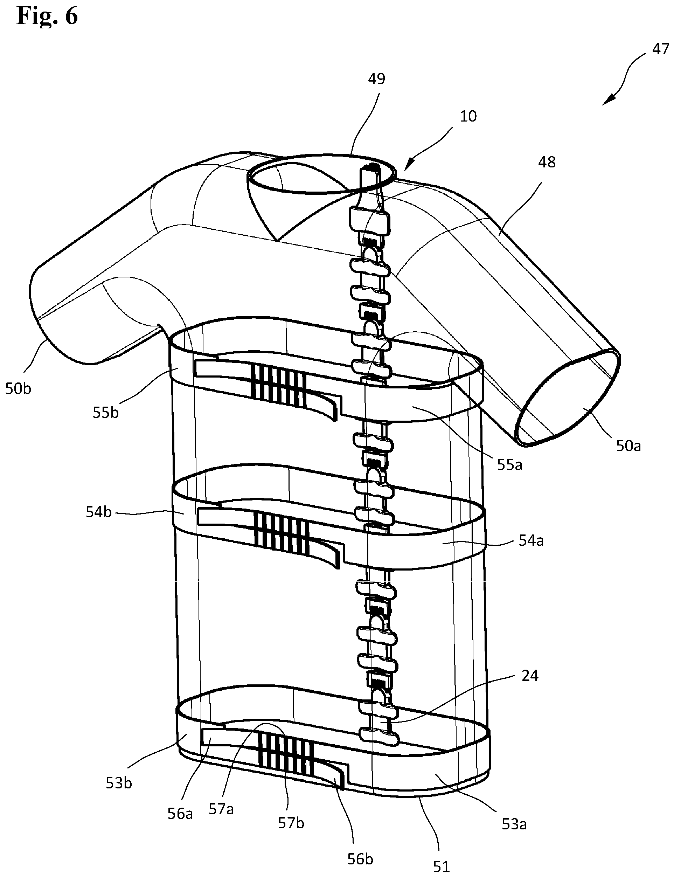

In Figur 6 ist eine perspektivische Vorderansicht auf das Bekleidungsstück 47 von Figur 5 gezeigt, wobei das Hemd 48 zum besseren Verständnis transparent dargestellt ist. Wie dort zu erkennen ist, umschließen die Paare von Bändern 53a, 53b, 54a, 54b, 55a, 55b den Brust- bzw. Baubereich des Hemds 48 komplett und überlappen sich an einer Vorderseite bzw. liegen dort jeweils seitlich aneinander an. Nachfolgend werden die Merkmale und die Funktion der Paare von Bändern am Beispiel des untersten Paars von Bändern 53a, 53b beschrieben.

Wie aus Figur 6 zu entnehmen ist, umschließen die Bänder 53a, 53b den Bauchbereich des Hemds 48 vollständig und die freien Enden 56a, 56b der Bänder 53a, 53b überlappen sich bzw. liegen seitlich aneinander an. Im Bereich der Enden 56a, 56b weisen die Bänder 53a, 53b eine Reihe von Markierungen 57a, 57b auf, welche in Längsrichtung der Bänder 56a, 56b voneinander beabstandet angeordnet sind.

Die Markierungen 57a, 57b kennzeichnen jeweils einen bestimmten Abstand zum entsprechenden Kettenglied auf der Rückseite des Hemds 48, an welchem die Bänder befestigt sind, und sind beispielsweise mittels Farben oder zugeordneter Zahlen unterscheidbar, jedoch auf beiden Bändern des Paars identisch ausgebildet. Hat die Testperson das Bekleidungsstück 47 angezogen, kann mittels der Paare von Bändern 53a, 53b, 54a, 54b, 55a, 55b die Gliederkette 11 der Messvorrichtung 10 durch die Testperson selbst exakt zur Wirbelsäule ausgerichtet werden. Hierzu zieht die Testperson beispielsweise an beiden Bändern 53a, 53b und bringt somit in Abhängigkeit des Bauchumfangs entsprechende identische Messmarkierungen an den Bändern 53a, 53b, hier beispielsweise die mit den Bezugszeichen 57a, 57b gekennzeichneten Messmarkierungen, zur Überlappung. Bei dem gezeigten Ausführungsbeispiel entsprechen die Messmarkierungen 57a, 57b jeweils der vierten Markierung der Bänder 53a, 53b ausgehend von dem achten Kettenglied 24. Folglich ist der Abstand von den Messmarkierungen 57a, 57b zu beiden Seiten um den Bauch der Testperson herum zu dem achten Kettenglied 24 identisch. Sofern die Messmarkierungen 57a, 57b nun nicht ohnehin schon mittig im Bauchbereich der Testperson angeordnet sind, kann die Testperson sehr einfach durch Ziehen der Bänder 53a, 53b die Messmarkierungen 57a, 57b an der Vorderseite mittig zum Bauch ausrichten, wodurch automatisch auch das achte Kettenglied 24 mittig zur Wirbelsäule ausgerichtet wird. Hierzu ist es vorteilhaft, wenn das Paar an Bändern 53a, 53b zumindest im Bereich von den Messmarkierungen 57a, 57b bis zum achten Kettenglied 24 anders als das Hemd 48 nicht elastisch, sondern in Längsrichtung fest ausgebildet ist.

Um eine einfache Fixierung der Paare von Bändern 53a, 53b, 54a, 54b, 55a, 55b an dem Hemd 48 nach erfolgter Zentrierung der Gliederkette 11 zu ermöglichen, kann an den Enden 56a, 56b der Bänder 53a, 53b jeweils ein Verbindungsmittel wie ein Klettverschluss zur Fixierung an einem korrespondierenden Klettband am Hemd 48 vorgesehen sein. Zudem können die Enden 56a, 56b der Bänder 53a, 53b bis zu den Messmarkierungen auch elastisch ausgebildet sein, um ein Bauch- und Brustbewegung der Testperson zu erleichtern.

Entsprechend sind auch die weiteren Paare von Bändern 54a, 54b, 55a, 55b ausgebildet, so dass über diese eine vollständige Zentrierung der Gliederkette 11 durch die Testperson vorgenommen werden kann und eine weitere Hilfsperson nicht notwendig ist. In einer alternativen Ausführungsform können auch mehrere oder weniger Paare von Bändern vorgesehen sein, um die Gliederkette 11 zu zentrieren. Insbesondere können durch entsprechende nicht dargestellte weitere Federelemente die dritten Drehgelenke der Kettenglieder, welche eine seitliche Drehung der Kettenglieder zueinander parallel zu einer Anlagefläche des Rückens der Testperson und damit eine Lateralflexion ermöglichen, in eine mittige Position beaufschlagt sein, so dass die Gliederkette zumindest in der Draufsicht von Figur 4 geradlinig vorgespannt ist. Durch diese federkraftbedingte Vorspannung ist die Gliederkette 11 geringfügig eigenstabil in Längsrichtung und kann auch durch wenige Paare von Bändern über die gesamte Länge seitlich verschoben und zur Wirbelsäule justiert werden.

Zur individuellen Anpassung einer Liegefläche, beispielsweise einer Matratze mit oder ohne zugeordnetem Lattenrost ist dann lediglich die Messvorrichtung 10 an der Testperson anzubringen, was beispielsweise durch eigenständiges Anziehen des Bekleidungsstücks 47 durch die Testperson erfolgend kann. Für den Fall, dass die Messvorrichtung 10 nicht optimal zur Wirbelsäule der Testperson positioniert ist, kann die Testperson optional die zuvor beschriebene Zentrierung und Justierung der Messvorrichtung 10 mittels der Paare von Bändern 53a, 53b, 54a, 54b, 55a, 55b vornehmen.

Anschließend muss sich die Testperson auf die Liegefläche legen und eine gewünschte Lage, beispielsweise eine Seitenlage wie in den Figuren 1 und 2 gezeigt, einnehmen. Mittels der Messvorrichtung 10 kann dann die Ist-Positionierung der Wirbelsäule einfach und schnell gemessen und beispielsweise an eine Auswerteeinheit und/oder eine Anzeigeeinheit übertragen werden. Die Auswerteeinheit kann aus den gemessenen Daten die Lage der Wirbelsäule berechnen und mit hinterlegten Daten vergleichen, so dass hieraus eine Handlungsempfehlung abgeleitet werden kann. Die Anzeigeeinheit kann beispielsweise ein 3D-Modell der Wirbelsäule in Echtzeit anzeigen und auf entsprechende Fehler in der Lagerung hinweisen. Hierauf aufbauend kann die Matratze durch Modifikation oder Austausch und/oder der Lattenrost durch Modifikation angepasst werden, um eine Soll-Positionierung der Wirbelsäule zu erreichen. Nach erfolgter Anpassung der Liegefläche kann der Erfolg nochmals über eine weitere Messung der Lage der Wirbelsäule der auf der Liegefläche ruhenden Person überprüft werden.

Die Soll-Positionierung der Wirbelsäule kann dabei aus hinterlegten Daten beispielsweise der medizinischen Fachliteratur entnommen werden, es kann jedoch auch vor der Ermittlung der Ist-Positionierung auf der Liegefläche die Lage der Wirbelsäule der stehenden Testperson gemessen werden, um die Soll-Positionierung entsprechend anzupassen.

Die beschriebene Messvorrichtung und das entsprechende Bekleidungsstück eignen sich auch zur Bestimmung der Positionierung der Wirbelsäule einer auf anderen Sitz-, Liege- und Schlafmöbeln ruhenden Testperson, beispielsweise zur Beurteilung und individuellen Anpassung und Einstellung eines Bürostuhls für eine Testperson. Durch die ganzheitliche Erfassung der Haltung des Oberkörpers können Fehlstellungen erkannt und verhindert werden. Zudem kann hierdurch auch die Körperhaltung aktiv über einen längeren Zeitraum insbesondere telemetrisch überwacht werden, beispielsweise beim Heben von Lasten, um risikoreiche Körperhaltungen und -bewegungen zu erkennen und vorzubeugen. Hierdurch kann eine ergonomische Arbeits- und Lebensweise unterstützt werden. Schließlich kann die Messvorrichtung sowie das Bekleidungsstück auch verwendet werden, um die Körperhaltung und -bewegungen für Virtual Reality-Anwendungen zu erfassen.

### Bezugszeichenliste

- 1: Testperson
- 2: Matratze
- 3: Kopf
- 4: Kopfkissen
- 5: Schlafsystem
- 6: Liegefläche
- 7: Gerade Wirbelsäule
- 8: Erste gekrümmte Wirbelsäule
- 9: Zweite gekrümmte Wirbelsäule
- 10: Messvorrichtung
- 11: Gliederkette
- 12: Wirbelsäule
- 13: Halswirbel
- 14: Brustwirbel
- 15: Lendenwirbel
- 16: Kreuzbein/Steißbein
- 17: Erstes Kettenglied
- 18: Zweites Kettenglied
- 19: Drittes Kettenglied
- 20: Viertes Kettenglied
- 21: Fünftes Kettenglied
- 22: Sechste Kettenglied
- 23: Siebtes Kettenglied
- 24: Achtes Kettenglied
- 25: Kopplungsabschnitt des zweiten Kettenglieds
- 26: Kopplungsabschnitt des siebten Kettenglieds
- 27: Grundkörper des zweiten Kettenglieds
- 28; 28b: Schubgelenk
- 29, 29b: Längensensor eines Kopplungsabschnitts
- 30, 30a, 30b: Erstes Drehgelenk eines Kopplungsabschnitts
- 31, 31a, 31b: Erstes Zwischenstück eines Kopplungsabschnitts
- 32, 32a, 32b: Erster Winkelsensor eines Kopplungsabschnitts
- 33, 33a, 33b: Zweites Drehgelenk eines Kopplungsabschnitts
- 34, 34a, 34b: Zweites Zwischenstück eines Kopplungsabschnitts
- 35, 35a, 35b: Zweiter Winkelsensor eines Kopplungsabschnitts
- 36, 36a, 36b: Federelement eines Kopplungsabschnitts
- 37, 37a, 37b: Drittes Drehgelenk eines Kopplungsabschnitts
- 38: Grundkörper des dritten Kettenglieds
- 39, 39a, 39b: Dritter Winkelsensor des zweiten Kettenglieds
- 40: Kopplungsabschnitt des ersten Kettenglieds
- 41: Grundkörper des ersten Kettenglieds
- 42: Schwerkraftsensor
- 43: Datenübertragungsmodul
- 44: Recheneinheit
- 45: Grundkörper des achten Kettenglieds
- 46: Energiespeicher
- 47: Bekleidungsstück
- 48: Hemd
- 49: Halsöffnung
- 50a, 50b: Armöffnung
- 51: Torsoöffnung
- 52: Justiervorrichtung
- 53a, 53b: Erster Paar von Bändern
- 54a, 54b: Zweites Paar von Bändern
- 55a, 55b: Drittes Paar von Bändern
- 56a, 56b: Enden der Bänder
- 57a, 57b: Markierungen

## Patentansprüche

1. Messvorrichtung (10) zur Bestimmung der Positionierung der menschlichen Wirbelsäule (12), mit einer Gliederkette (11) umfassend mehrere gelenkig verbundene Kettenglieder (18; 19; 20; 21; 22; 23), wobei jedes Kettenglied (18; 19; 20; 21; 22; 23) einen Kopplungsabschnitt (25; 26) zur Verbindung mit einem benachbarten Kettenglied (18; 19; 20; 21; 22; 23) aufweist und jeder Kupplungsabschnitt (25; 26) jeweils mehrere Gelenke (28; 28b; 30; 30a; 30b; 33; 33a; 33b; 37; 37a; 37b) mit Winkel- und/oder Längensensoren (29; 29a; 29b; 32; 32a; 32b; 35; 35a; 35b; 39; 39a; 39b) zur Bestimmung der relativen gegenseitigen Position benachbarter Kettenglieder (18; 19; 20; 21; 22; 23) umfasst,
**gekennzeichnet durch**
einen Schwerkraftsensor (42) zur Bestimmung der räumlichen Orientierung.

2. Messvorrichtung (10) nach Anspruch 1, **dadurch gekennzeichnet, dass** jeder Kopplungsabschnitt (18; 19; 20; 21; 22; 23) jeweils drei Drehgelenke (30; 33; 37; 30a; 33a; 37a; 30b; 33b; 37b) mit zugeordneten Winkelsensoren (32; 35; 39; 32a; 35a; 39a; 32b; 35b; 39b) aufweist.

3. Messvorrichtung (10) nach Anspruch 2, **dadurch gekennzeichnet, dass** jeder Kopplungsabschnitt (25; 26) zusätzlich ein Schubgelenk (28; 28b) mit einem Längensensor (29; 29b) aufweist.

4. Messvorrichtung (10) nach einem der Ansprüche 2 oder 3, **dadurch gekennzeichnet, dass** ein Drehgelenk (33; 33a; 33b) jedes Kopplungsabschnitts (25; 26) mittels eines Federelements (36; 36a; 36b) vorgespannt ist.

5. Messvorrichtung (10) nach einem der vorhergehenden Ansprüche, **gekennzeichnet durch** ein Datenübertragungsmodul (43), insbesondere ein Funk-Übertragungsmodul, zur Übertragung der Daten der Sensoren (29; 29b; 32; 32a; 32b; 35; 35a; 35b; 39; 39a; 39b) von der Gliederkette (11) an eine Anzeigeeinheit.

6. Bekleidungsstück (47) zur Messung der Positionierung des menschlichen Oberkörpers mit einem textilen Grundkörper (48), **dadurch gekennzeichnet, dass** an dem Grundkörper (48) eine Messvorrichtung (10) nach einem der Ansprüche 1 bis 5 befestigt ist.

7. Vorrichtung zur individuellen Anpassung einer Liegefläche umfassend ein anpassbares Schlafsystem, eine Auswerteeinheit und eine Messvorrichtung (10), **dadurch gekennzeichnet, dass** die Messvorrichtung (10) nach einem der Ansprüche 1 bis 5 ausgebildet ist.

8. Vorrichtung nach Anspruch 7, **dadurch gekennzeichnet, dass** die Messvorrichtung (10) in ein Bekleidungsstück (47) integriert ist.

9. Verfahren zur individuellen Anpassung einer Liegefläche, **gekennzeichnet durch** die Schritte:
- Anbringen einer Messvorrichtung (10) nach einem der Ansprüche 1 bis 5 an einer Testperson;
- Ermittlung der Ist-Positionierung der Wirbelsäule der auf der Liegefläche ruhenden Testperson mittels der Messvorrichtung (10);
- Auswahl und Anpassung der Liegefläche in Abhängigkeit einer Soll-Positionierung der Wirbelsäule.

## Claims

1. A measuring device (10) for determining the positioning of the human spine (12), comprising a link chain (11) including a plurality of articulately connected chain links (18; 19; 20; 21; 22; 23), each chain link (18; 19; 20; 21; 22; 23) having a coupling portion (25; 26) for connection to an adjacent chain link (18; 19; 20; 21; 22; 23), each coupling portion (25; 26) having a plurality of joints (28; 28b; 30; 30a; 30b; 33; 33a; 33b; 37; 37a; 37b) with angle and/or length sensors (29; 29a; 29b; 32; 32a; 32b; 35; 35a; 35b; 39; 39a; 39b) for determining the relative mutual position of neighboring chain links (18; 19; 20; 21; 22; 23),
**characterized by**
a gravity sensor (42) for determining the spatial orientation.

2. Measuring device (10) according to claim 1, **characterized in that** each coupling section (18; 19; 20; 21; 22; 23) has three swivel joints (30; 33; 37; 30a; 33a; 37a; 30b; 33b; 37b) with associated angle sensors (32; 35; 39; 32a; 35a; 39a; 32b; 35b; 39b).

3. Measuring device (10) according to claim 2, **characterized in that** each coupling section (25; 26) additionally has a slide joint (28; 28b) with a length sensor (29; 29b).

4. Measuring device (10) according to one of claims 2 or 3, **characterized in that** a swivel joint (33; 33a; 33b) of each coupling section (25; 26) is preloaded by means of a spring element (36; 36a; 36b).

5. Measuring device (10) according to one of the preceding claims, **characterized by** a data transmission module (43), in particular a radio transmission module, for transmitting the data of the sensors (29; 29b; 32; 32a; 32b; 35; 35a; 35b; 39; 39a; 39b) from the link chain (11) to a display unit.

6. Garment (47) for measuring the positioning of the human upper body with a textile base body (48), **characterized in that** a measuring device (10) according to one of claims 1 to 5 is attached to the base body (48).

7. Device for the individual adjustment of a lying surface, comprising an adjustable sleeping system, an evaluation unit and a measuring device (10), **characterized in that** the measuring device (10) is designed according to one of claims 1 to 5.

8. Device according to claim 7, **characterized in that** the measuring device (10) is integrated into a garment (47).

9. Method for the individual adjustment of a lying surface, **characterized by** the following steps:
- attaching a measuring device (10) according to one of claims 1 to 5 to a test person;
- determining the actual positioning of the spine of the test person resting on the lying surface by means of the measuring device (10);
- selecting and adjusting the lying surface as a function of a target positioning of the spine.

## Revendications

1. Dispositif de mesure (10) pour déterminer le positionnement de la colonne vertébrale humaine (12), avec une chaîne à maillons (11) comprenant plusieurs maillons de chaîne (18; 19; 20; 21; 22; 23) reliés de manière articulée, chaque maillon de chaîne (18; 19; 20; 21; 22; 23) présentant une section d'accouplement (25; 26) pour la liaison avec un maillon de chaîne voisin (18; 19; 20; 21; 22; 23) et chaque section d'accouplement (25; 26) comprend respectivement plusieurs articulations (28; 28b; 30; 30a; 30b; 33; 33a; 33b; 37; 37a; 37b) avec des capteurs d'angle et/ou de longueur (29; 29a; 29b; 32; 32a; 32b; 35; 35a; 35b; 39; 39a; 39b) pour déterminer la position mutuelle relative de maillons de chaîne voisins (18; 19; 20; 21; 22; 23),
**caractérisé par**
un capteur de gravité (42) pour déterminer l'orientation spatiale.

2. Dispositif de mesure (10) selon la revendication 1, **caractérisé en ce que** chaque section de couplage (18; 19; 20; 21; 22; 23) présente respectivement trois articulations rotative (30; 33; 37; 30a; 33a; 37a; 30b; 33b; 37b) avec des capteurs angulaires associés (32; 35; 39; 32a; 35a; 39a; 32b; 35b; 39b).

3. Dispositif de mesure (10) selon la revendication 2, **caractérisé en ce que** chaque section de couplage (25; 26) comprend en outre une articulation de poussée (28; 28b) avec un capteur de longueur (29; 29b).

4. Dispositif de mesure (10) selon l'une des revendications 2 ou 3, **caractérisé en ce qu'**une articulation rotative (33; 33a; 33b) de chaque section de couplage (25; 26) est précontrainte au moyen d'un élément élastique (36; 36a; 36b).

5. Dispositif de mesure (10) selon l'une des revendications précédentes, **caractérisé par** un module de transmission de données (43), notamment un module de transmission radio, pour transmettre les données des capteurs (29; 29b; 32; 32a; 32b; 35; 35a; 35b; 39; 39a; 39b) de la chaîne à maillons (11) à une unité d'affichage.

6. Pièce de vêtement (47) pour mesurer le positionnement du torse humain avec un corps de base textile (48), **caractérisée en ce qu'**un dispositif de mesure (10) selon l'une des revendications 1 à 5 est fixé sur le corps de base (48).

7. Dispositif d'adaptation individuelle d'une surface de couchage comprenant un système de couchage adaptable, une unité d'évaluation et un dispositif de mesure (10), **caractérisé en ce que** le dispositif de mesure (10) est conçu selon l'une des revendications 1 à 5.

8. Dispositif selon la revendication 7, **caractérisé en ce que** le dispositif de mesure (10) est intégré dans un vêtement (47).

9. Procédé d'adaptation individuelle d'une surface de couchage, **caractérisé par** les étapes suivantes :
- Mise en place d'un dispositif de mesure (10) selon l'une des revendications 1 à 5 sur une personne test;
- Détermination du positionnement réel de la colonne vertébrale de la personne test reposant sur la surface de couchage au moyen du dispositif de mesure (10);
- Sélection et adaptation de la surface de couchage en fonction d'un positionnement théorique de la colonne vertébrale.
